# EUROPÄISCHE PATENTSCHRIFT

(11) **EP 0 862 577 B1**
(45) Veröffentlichungstag und Bekanntmachung des Hinweises auf die Patenterteilung: **14.04.1999**
(21) Anmeldenummer: 96945140.0
(22) Anmeldetag: 19.09.1996
(51) Int. Cl.: C07J 41/00, A61K 31/565

(54) **SULFAMAT-DERIVATE VON 1,3,5(10)-ESTRATRIEN-DERIVATEN, VERFAHREN ZU IHRER HERSTELLUNG UND DIESE VERBINDUNGEN ENTHALTENDE PHARMAZEUTISCHE ZUSAMMENSETZUNGEN**
SULPHAMATE DERIVATIVES OF 1,3,5(10)-ESTRATRIENE DERIVATIVES, METHODS OF PRODUCING THEM AND PHARMACEUTICAL COMPOUNDS CONTAINING THEM
DERIVES SULFAMATE DE DERIVES 1,3,5(10)-ESTRATRIENE, PROCEDE DE PRODUCTION CORRESPONDANT ET COMPOSITIONS PHARMACEUTIQUES RENFERMANT CES COMPOSES

(30) Priorität: 19.10.1995 DE 19540233
(43) Veröffentlichungstag der Anmeldung: 09.09.1998
(73) Patentinhaber: Jenapharm GmbH & Co. KG, 07745 Jena (DE)
(72) Erfinder: SCHWARZ, Sigfrid, D-07743 Jena (DE); ELGER, Walter, D-14195 Berlin (DE); REDDERSEN, Gudrun, D-07749 Jena (DE); SCHNEIDER, Birgitt, D-07745 Jena (DE); THIEME, Ina, D-07616 Graitschen (DE); RICHTER, Margit, D-07745 Jena (DE)
(74) Vertreter: Wablat, Wolfgang, Dr.Dr.
(86) Internationale Anmeldenummer: DE9601823
(87) Internationale Veröffentlichungsnummer: WO9714712

(56) Entgegenhaltungen:
- DE-A- 1 949 095
- FR-A- 2 133 484
- PHARMAZIE, Bd. 30, Nr. 1, Januar 1975, BERLIN DE, Seiten 52-53, XP002026377 W. STÖLZNER ET AL: "Untersuchungen zur Dissoziation zwischen antigonadatrophen und interzeptiven Aktivitäten ausgewählter Östratrienderivate"
- PHARMAZIE, Bd. 30, Nr. 1, Januar 1975, BERLIN DE, Seiten 17-21, XP002026378 S. SCHWARZ ET AL: "Steroide 15. Mitteilung: Sulfonyloxyderivate von Östrogenen"
- JOURNAL OF STEROID BIOCHEMISTRY AND MOLECULAR BIOLOGY , Bd. 55, Nr. 3/4, 29.Dezember 1995, Seiten 395-403, XP002026379 W. ELGER ET AL: "Sulfamates of Various Estrogens are Prodrugs with Increased Systemic and Reduced Hepatic Estrogenicity at Oral Application"

## Beschreibung

Die Erfindung betrifft neue Sulfamat-Derivate von 1,3,5(10)-Estratrien-Derivaten, Verfahren zu ihrer Herstellung und diese Verbindungen enthaltende pharmazeutische Zusammensetzungen.

Estrogene spielen in der hormonalen Kontrazeption und in der klimakterischen Hormon-Replacement-Therapie (HRT) sowie bei der Behandlung gynaekologischer (z. B. Mammacarcinom) und andrologischer (z. B. Prostatacarcinom) Krankheitsbilder eine wesentliche Rolle. In der HRT und für die Kontrazeption werden Estrogene ganz überwiegend in Kombination mit einem Gestagen eingesetzt, z. B. Levonorgestrel, Desogestrel, Norethisteron, Cyproteronazetat, Chlormadinonazetat, Dienogest.

Im Falle der Kontrazeption werden Estrogene einmal dazu benötigt, um Follikelreifung und Ovulation sicher zu unterdrücken, andererseits substituieren sie dann die weitgehend unterdrückte endogene, ovarielle Sekretion von Estradiol. Diese Substitution ist wesentlich für die Erhaltung eines artifiziellen Menstruationszyklus und anderer Funktionen der Sexualorgane, die mit einem Gestagen allein nicht befriedigend gelingt. Daneben haben endogene und exogene Estrogene wichtige zentralnervöse und metabolische Funktionen im weiblichen Organismus: Normale Estrogenspiegel tragen zum Wohlbefinden entscheidend bei. Ihre Anwesenheit wirkt dem Entstehen von Herz-Kreislauf-Erkrankungen über verschiedene Mechanismen entgegen: Erzeugung von "günstigen" Lipoproteinmustern im Blut, Hemmung der Lipideinlagerung in der Gefäßwand, Senkung des Blutdrucks durch günstige Beeinflussung des Gefäßtonus, Reduktion des Perfusionswiderstandes in wichtigen Gefäßgebieten, Dämpfung kontraktiler Reize am Gefäßmuskel. Unter der Wirkung von Estrogenen setzen die Gefäßinnenwände Faktoren frei, die der Entstehung von Blutgerinnseln entgegen wirken. Estrogene sind bei der Frau zur Erhaltung der Knochenstruktur unerläßlich. Ihr Verlust kann die Entwicklung eines Knochenabbaus (Osteoporose) bewirken. Die letztgenannten "zentralnervösen" und "metabolischen" Effekte der Estrogene sind wesentlicher Gesichtspunkt der HRT. Es kann als erwiesen gelten, daß Estrogene im Organismus des Mannes analoge Funktionen besitzen, und ihr Entzug zu ähnlichen Störungen führt wie bei der Frau. Lediglich der Umstand, daß die männliche Hormonproduktion weniger regelhaft und in höherem Lebensalter versiegt als bei der Frau bedingt einen Geschlechtsunterschied.

Bei allen positiven Aspekten der Estrogentherapie gibt es ungelöste Probleme, die die therapeutische Anwendung von Estrogenen einschränken oder unerwünschte Wirkungen beinhalten:

Die bekannten Estrogene weisen pharmakokinetische Defizite auf. Natürliche Estrogene (Estradiol, Estron, Estronsulfat, Ester von Estradiol, Estriol) werden bei oraler Anwendung nur zum geringsten Teil bioverfügbar. Dieser Anteil ist individuell so variabel, daß generelle Dosisempfehlungen nicht möglich sind. Problematisch ist auch die rasche Eliminierung der Substanzen aus dem Blut. Die Estrogensubstitution in der HRT muß sehr oft individuell angepaßt werden.

Entsprechendes gilt für synthetische Estrogene. Das wichtigste synthetisch abgewandelte estrogene Steroid ist das Ethinylestradiol (EE). Dieses Estrogen ist beherrschend in der oralen hormonalen Kontrazeption. Neben EE wird in wenigen Produkten das Mestranol eingesetzt, das ein "Prodrug" ist und im Organismus zu EE verstoffwechselt wird. EE ist bei oraler Applikation (Mensch) viel besser bioverfügbar als die o. g. natürlichen Estrogene, allerdings variiert die orale Bioverfügbarkeit individuell außerordentlich stark. Verschiedene Autoren haben auf diesen Umstand und das z. T. regellose Verhalten der Blutspiegelverläufe nach oraler Applikation dieser Substanz hingewiesen (Goldzieher, J. W. 1989, Goldzieher, J. W. 1990, Hümpel, M. 1987, Kuhnz, 1993).

Daneben weisen die bekannten Estrogene auch pharmakodynamische Defizite auf. Bei oraler Anwendung gelangen Wirkstoffe nach Resorption aus dem Darmlumen über die Leber in den Organismus. Für estrogene Wirkstoffe ist diese Tatsache von besonderer Bedeutung, da die Leber ein Erfolgsorgan für Estrogene ist und deren orale Gabe und die damit verbundene Leberpassage zu starken Estrogeneffekten in der Leber führt. Zu den Sekretionsaktivitäten, die in der menschlichen Leber durch Estrogene reguliert werden, gehören u. a. die Synthesen der Transportproteine CBG, SHBG, TBG, das Angiotensinogen, verschiedene Faktoren, die in der Physiologie der Blutgerinnung eine wichtige Rolle spielen und die Lipoproteine. Werden dem weiblichen Organismus natürliche Estrogene unter Umgehung der Leberpassage zugeführt, z. B. durch transdermale Applikation, so bleiben die genannten Leberfunktionen praktisch unverändert. Therapeutisch aequivalente Dosen natürlicher Estrogene (Definition s. o.) führen bei oraler Applikation zu deutlichen Reaktionen hepatischer Parameter: Anstieg von SHBG, CBG, Angiotensinogen, HDL (high density lipoproteins). Deutlich stärker ausgeprägt als bei natürlichen Estrogenen sind entsprechende hepatische Estrogeneffekte bei equinen Estrogenmischungen, sog. konjugierte Estrogene (Campbell, S. und Mitarb., 1981). Noch stärkere hepatische Estrogenität besitzen das Ethinylestradiol und das DES.

Bezogen auf antigonadotrope Eigenschaften ist das EE in der Leber ca 4-18 mal stärker Estrogen wirksam als oral verabreichte natürliche Estrogene (Campbell, S. und Mitarb., 1981). Es liegt also eine sehr ungünstige Dissoziation von Eigenschaften vor.

Estrogenisch N-Alkylierte Estrogen-3-sulfamate sind von FR-A-2,133,484 und "Die Pharmazie" Band 30(1) S. 17-21 und S. 52-53 bekannt.

Diese Defizite haben eine erhebliche klinische Bedeutung für die Verwendung der bekannten natürlichen und synthetischen Estrogene.

Im Falle der Estrogentherapie mit hoch dosierten Estrogenen bei Männern mit Prostatacarcinom sind thromboembolische Erkrankungen mit tödlichem Ausgang eine bekannte Komplikation. In abgeschwächter Form bestimmt das Nebenwirkungspotential von herkömmlichen Estrogenen in der Leber die Strategie der oralen hormonalen Kontrazeption. Im Hinblick auf erwünschte kontrazeptive Effekte sowie die Erhaltung des monatlichen Menstruationsgeschehens, einerseits, und die Beachtung eines erheblichen Potentials von Nebenwirkungen, andererseits, ist die schwere Steuerbarkeit der erwünschten Blutspiegel von EE ein großes Problem im Sinne einer Gratwanderung. Möglicherweise kann ein erheblicher Prozentsatz von Frauen orale Kontrazeptiva nicht anwenden, weil entweder Blutungsanomalien oder estrogenbedingte Nebenwirkungen die Akzeptanzgrenze überschreiten.

Die Hormontherapie mit natürlichen Hormonen erfordert mit der heutigen Technologie durchweg individuelle Dosisanpassungen. Entsprechende Behandlungen sind mit großen Unsicherheiten behaftet und beinhalten konkret die Gefahr von Über- und Unterdosierung.

Der vorliegenden Erfindung liegt daher die Aufgabe zugrunde, neue estrogen wirksame Verbindungen zu schaffen, welche die aufgezeigten Nachteile vermeiden.

Diese Aufgabe wird erfindungsgemäß durch die Schaffung neuer Sulfamat-Derivate der allgemeinen Formel I 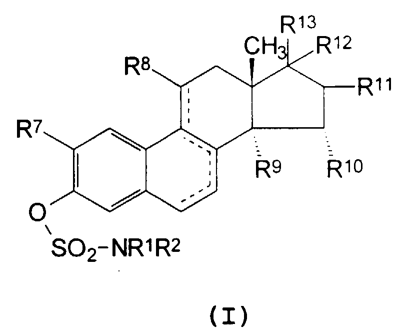 worin
R¹ eine -CO-R³-, -CO-OR⁴-, -CO-NR⁵R⁶- -SO₂-R⁴ oder -SO₂-NR⁵R⁶-Gruppe ist,
   worin
   R³ ein Wasserstoffatom ist oder die Bedeutung von R⁴ hat,
   R⁴ einen C₁-C₅-Alkyl-, einen C₂-C₅-Alkenylrest, einen C₃-C₆-Cycloalkylrest oder einen Arylrest mit bis zu 9 Kohlenstoffatomen darstellt,
   R⁵ und R⁶ unabhängig voneinander ein Wasserstoffatom, einen C₁-C₅-Alkylrest, einen Arylrest mit bis zu 9 Kohlenstoffatomen oder zusammen mit dem N-Atom einen Polymethyleniminorest mit 3 bis 6 Kohlenstoffatomen oder einen Morpholinorest bedeuten,
R² ein Wasserstoffatom oder ein physiologisch verträgliches Metall ist oder die Bedeutung von R⁴ hat,
R⁷ und R⁸ unabhängig voneinander ein Wasserstoffatom, eine Hydroxygruppe oder einen C₁-C₅-Alkoxyrest darstellen,
R⁹ und R¹⁰ jeweils ein Wasserstoffatom oder zusammen eine Methylengruppe bedeuten,
R¹¹, R¹² und R¹³ unabhängig voneinander ein Wasserstoffatom oder eine Hydroxygruppe, welche gegebenenfalls mit physiologisch verträglichen anorganischen oder organischen Säuren verestert ist, darstellen, oder R¹² oder R¹³ ein Alkinylrest mit bis zu 5 Kohlenstoffatomen ist,
die Ringe B und C gegebenenfalls eine oder zwei Doppelbindungen enthalten
und R⁸, R¹¹ und R¹² unabhängig voneinander α- oder β-ständig angeordnet sind,
   gelöst.

Physiologisch verträgliche Metalle, welche am Rest R² vorhanden sein können, sind beispielsweise Alkali- oder Erdalkalimetalle. Besonders bevorzugt sind Natrium und Kalium.

Übliche physiologisch verträgliche anorganische und organische Säuren, mit denen die Hydroxygruppen der Reste R¹¹, R¹² und R¹³ verestert sein können, sind beispielsweise Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure.

Weitere verwendbare Säuren sind beispielweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die erfindungsgemäßen Verbindungen übertreffen Ethinylestradiol hinsichtlich systemischer oraler estrogener Wirksamkeit. Sie weisen eine günstigere Relation erwünschter und therapeutisch unerwünschter Effekte auf. Bei maximalen Estrogeneffekten in Uterus beziehungsweise Vagina sind diese Substanzen in der Leber nicht stärker estrogen als natürliche Estrogene bei parenteraler Applikation in systemisch aequipotenter Dosierung. Durch diese Konstellation werden mit den erfindungsgemäßen neuen, estrogen wirksamen Verbindungen Vorteile im Vergleich zu allen heute in der oralen Therapie eingesetzten oder bekannten natürlichen und synthetischen Estrogenen erreicht.

Es wurde überraschenderweise gefunden, daß eine Acylierung, Sulfonierung oder Amidosulfonierung der 3-Sulfamat-Grußße von 1,3,5(10)-Estratrien-Derivaten, im Gegensatz zur Alkylierung, die systemische estrogene Wirkung nicht abschächt, sondern verstärkt. Die für die erfindungsgemäßen Verbindungen nachgewiesene geringe estrogene Wirkung auf hepatische Estrogenparameter im Vergleich zur starken systemischen Aktivität ist ein zusätzlicher Vorteil der erfindungsgemäßen Verbindungen in Relation zu den heute in der oralen Therapie eingesetzten natürlichen und synthetischen Östrogene.

Der Nachweis der vorteilhaften Eigenschaften der erfindungsgemäßen Verbindungen erfolgte durch tierexperimentelle Methoden:
1. Induktion von Uteruswachstum und Vaginalverhornung nach einmaliger oraler Applikation bei ovariektomierten Ratten (Allen-Doisy Test) : Test zur Quantifizierung systemischer Estrogenwirkung.
2. Erfassung systemischer und hepatischer Estrogenwirkungen bei ovariektomierten Ratten unter und nach 7-tägiger Behandlung: Test zur Bestimmung der Relation von systemischer und hepatischer Estrogenwirkung.

Im Allen-Doisy-Test erfolgte eine einmalige Behandlung mit Testsubstanz beziehungsweise Vehikel an Tagl (=d1). Beobachtung und Versuch endeten an Tag 4 (= d4). Bis dahin wurde die Vaginalcytologie täglich kontrolliert. Bei Versuchsende wurden die Uterusgewichte ermittelt.

Im Test auf systemische und hepatische estrogene Aktivität war der Behandlungsbeginn definiert als Tag 1 (= d1), Behandlungsende war Tag 7 (= d7). Tag 8 wurden die Tiere getötet, verschiedene Organe (Uteri, Nebennieren, Leber) entnommen und gewogen. Blut wurde in Aethernarkose vor der Behandlung (= d0) bzw. d4 und d8 aus dem retrobulbären Plexus entnommen. Im gewonnenen Serum wurden Angiotensin I, Cholesterin, HDL-Cholesterin und andere Faktoren bestimmt. Angiotensin I ist ein Parameter für direkte Estrogenwirkungen in der Leber, dies trifft auch für Gesamtcholesterin und HDL-Cholesterin zu.

### Bestimmungsmethoden:

*Angiotensin -* Modifizierter RIA für Reninaktivität, Firma Sorin;
*Cholesterin/HDL -* enzymatische Tests, photometrische Bestimmung, Reagentien Firma Dr. Bruno Lange GmbH.

### Ergebnisdarstellung:

1.Systemische Estrogenwirkung:
   Angabe der Dosierung, bei der ein vaginaler Oestrus induziert wird,
2. Hepatische Estrogenwirkung:
   Dosierung, die im Vergleich zu ovariektomierten Kontrollen Uterusgewichte verdoppelt, Dosierungen, die 50% Anstieg, bzw.Abfall bestimmter Parameter im Vergleich zu Kontrollen oder zum Vorwert bewirken.

### Versuchstiere:

Adulte weibliche Wistar-Ratten (Züchter: Fa Tierzucht Schönwalde GmbH) wurden ovariektomiert. Zwei Wochen später begann die Behandlung. Angegebene Dosierungen beziehen sich bei Estern auf den Steroidanteil der Substanzen. Die Zuordnung der einzelnen Tiere zu ihren Gruppen erfolgte durch Randomisierung.

Die tierexperimentellen Ergebnisse sind in den Tabellen 1 und 2 dargestellt:
1. Systemische estrogene Aktivität
In Tabelle 1 wird nachgewiesen, daß Acylsulfamate von Estron und Estradiol bei niedrigeren Dosierungen eine 100%ige Vaginalverhornung auslösen als die jeweiligen Mutterestrogene. Selbst Ethinylestradiol wird von diesen Verbindungen übertroffen. Die stärkere systemische Aktivität der Acylsulfamate wird auch ersichtlich, wenn man das Uteruswachstum unter den getesteten Estrogenen vergleicht. Aus Tabelle 1 wird ersichtlich, daß Acylsulfamate Uteruswachstum stärker stimulieren als die mituntersuchten Referenzestrogene.

**Tabelle 1:**

| Versuchsgruppen, Tierzahlen ¹⁾ | | | | | | |
|---|---|---|---|---|---|---|
| **Substanzen** | **Dosierungen (µg/Tier, p.o.)** | | | | | |
| | 0,1 | 1,0 | 10,0 | 100, 0 | 300, 0 | 1000,0 |
| E1 (Estron) | | | | 0/5 | 0/5 | 2/5 |
| E2 (Estradiol) | | | | 0/5 | **5/5** | 5/5 |
| EE (Ethinylestradiol) | 0/7 | 3/7 | 2/7 | **7/7** | | |
| J 1045 | 0/5 | 0/5 | **5/5** | 5/5 | | |
| J 1046 | 0/5 | 0/5 | 3/5 | **5/5** | | |

| | | | | | | |
|---|---|---|---|---|---|---|
| ¹⁾ ausgewertete Tiere bei Versuchsende | | | | | | |

2. Hepatische estrogene Aktivität
In Tabelle 2 sind die Dosierungen für die untersuchten Substanzen angegeben, die im Vergleich zur Kontrollgruppe die Uterusgewichte verdoppelten bzw. zu einer 50%igen Änderung der Blutspiegel von Angiotensin-1 und HDL-Cholesterin führten. Unter Ethinylestradiol liegt der so definierte Wert hepatischer Aktivität unter der Dosis, die die Uterusgewichte verdoppelt. Zum Teil gilt diese Feststellung auch für die untersuchten natürlichen Estrogene. Acylsulfamatestrogene zeigen hepatische Aktivität gemäß Definition nur bei Dosierungen weit oberhalb uteruswirksamer Bereiche.

**Tabelle 2**

| Relative uterine und hepatische Estrogenwirkung von J 1045 | | | | | |
|---|---|---|---|---|---|
| | **Äquipotente orale Dosis (µg / Tier / Tag)** | | | | |
| Substanz | Uterus Δ 100 % ↑ | Angiotensin \| Δ 50 % ↑ | HDL-Cholesterol Δ 50 % ↓ | Quotient von A \| Δ 50 % ↑ und Uterus Δ 100 % ↑ | Quotient von HDL-Cholesterol Δ 50 % ↓ und Uterus Δ 100 % ↑ |
| EE | 15 | 6,5 | 3 | 0,43 | 0,2 |
| E1 | 170 | 340 | 210 | 2 | 1 |
| E2 | 240 | 230 | 95 | 1 | 0,4 |
| J 1045 | 4 | > 100 500 (extrapol.) | 15 | > 25 125 (extrapol.) | 4 |

Wie die Tabellen 1 und 2 zeigen, haben die erfindungsgemäßen Derivate natürlicher und synthetischer Estrogene in Relation zu ihren Mutterestrogenen eine stark erhöhte sytemische estrogene Wirkung. Dadurch werden die therapeutischen Effekte leichter erreicht, d.h. mit geringeren Dosen, als mit der oralen Anwendung der Mutterestrogene. Eine Reduktion unerwünschter Stoffwechseleffekte ergibt sich aus der beobachteten Senkung der hepatischen Estrogenwirkungen in Relation zur erhöhten systemischen Aktivität.

Ein weiterer Gegenstand der Erfindung ist ein Verfahren zur Herstellung der Sulfamat-Derivate der allgemeinen Formel I 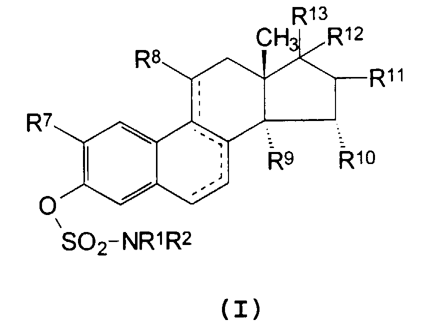 worin die Reste R¹ bis R¹³ die oben angegebene Bedeutung haben,
indem man in an sich bekannter Weise
a) Estra-1,3,5(10)-trien-3-yl-sulfamat-Derivate, welche am Stickstoffatom des Sulfamatrestes mindestens ein Wasserstoffatom tragen, mit einer aktivierten Carbonsäure, Carbamidsäure, Sulfonsäure oder Amidosulfonsäure
   oder
b) 3-Hydroxy-estra-1,3,5(10) -trien-Derivate mit einer aktivierten N-Acyl-amidosulfonsäure, N-Sulfonylamidosulfonsäure oder N-Amidosulfonyl-amidosulfonsäure
jeweils gegebenenfalls in Gegenwart einer Base umsetzt, die so erhaltenen Produkte gegebenenfalls in geeigneter Weise weiter umsetzt und gegebenenfalls die so erhaltenen Produkte in physiologisch verträgliche Metallsalze überführt.

Ein weiterer Gegenstand der vorliegenden Erfindung sind pharmazeutische Zusammensetzungen, enthaltend mindestens ein Sulfamat-Derivat der allgemeinen Formel I 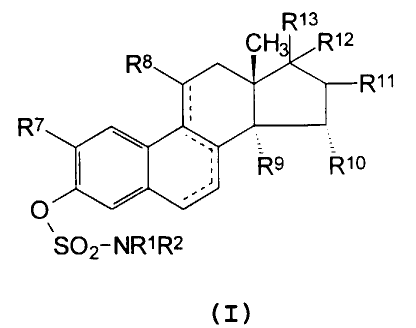 worin die Reste R¹ bis R¹³ die oben angegebene Bedeutung haben, gegebenenfalls zusammen mit pharmazeutisch verträglichen Hilfs- und Trägerstoffen.

Gegenstand der vorliegenden Erfindung sind auch pharmazeutische Zusammensetzungen und Arzneimittel zur oralen, rektalen, vaginalen, subcutanen, percutanen, intravenösen oder intramuskulären Applikation, die neben üblichen Träger- und Verdünnungsmitteln eine Verbindung der allgemeinen Formel I enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien und Mittel zu vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die folgenden Beispiele erläutern die Erfindung:

### Beispiel 1

### 17-Oxo-estra-1,3,5(10) -trien-3-yl-(N-acetyl)sulfamat (J 1046)

Estronsulfamat (2,0 g) wurde in Pyridin gelöst. Man gab zu dieser Lösung Acetanhydrid (100 ml) und rührte das Gemisch 2 Stunden bei +23 °C. Danach wurde mit Eis zersetzt, der Niederschlag abfiltriert, mit Wasser neutral gewaschen und im Luftstrom getrocknet. Umkristallisation aus Aceton ergab die Titelverbindung. Fp.: 218-223 °C (Aceton)

### Beispiel 2

### 17β-Hydroxy-estra-1,3,5(10)-trien-3-yl-(N-butyryl)sulfamat

Estronsulfamat (1,3 g) wurde in einem Gemisch aus Dichlormethan (45 ml) und Triethylamin (0,5 ml) gelöst. Man fügte unter Rühren bei +23 °C p-Dimethylaminopyridin (0,455 g) und Buttersäureanhydrid (12 ml) hinzu. Nach 20stündigem Rühren bei +23 °C wurde die Reaktionslösung 5mal mit Wasser (je 70 ml) gewaschen, über wasserfreiem Natriumsulfat getrocknet und im Vakuumrotationsverdampfer eingeengt. Den Rückstand versetzte man mit n-Hexan (50 ml), worauf Kristallisation einsetzte. Ein Teil der abfiltrierten Kristalle (0,9 g), die 17-Oxo-estra-1,3,5(10)-trien-3-yl-(N-butyryl)sulfamat darstellten, löste man in einem Gemisch aus Tetrahydrofuran (36 ml) und Methanol (36 ml). Zu der auf +5 °C abgekühlten Lösung gab man unter Rühren Natriumborhydrid (0,36 g). Nach Beendigung der Reduktion (DC-Kontrolle) wurde das Gemisch mit Essigsäure neutralisiert und das Produkt mit Wasser gefällt. Umkristallisation aus Aceton/n-Hexan ergab die Titelverbindung.
Fp.: 197-201 °C (Aceton/n-Hexan)

### Beispiel 3

### 17-Oxo-estra-1,3,5(10)-trien-3-yl-(N-propionyl)sulfamat

Zu einer Lösung von Estronsulfamat (1,0 g) in Dichlormethan (35 ml) gab man nacheinander Triethylamin (0,4 ml), p-Dimethylaminopyridin (0,35 g) und Propionsäureanhydrid (7,4 ml). Man rührte das Reaktionsgemisch 20 Stunden bei +23 °C, dann wurde mit Eis zersetzt. Die organische Phase wurde mit gesättigter wäßriger Natriumhydrogencarbonatlösung und mit Wasser gewaschen, über wasserfreiem Natriumsulfat getrocknet und im Vakuumrotationsverdampfer eingeengt, wobei man die Titelverbindung erhielt.
Fp.: 209-211 °C

### Beispiel 4

### 17-Oxo-estra-1,3,5(10)-trien-3-yl-(N-t-butoxycarbonyl)sulfamat

Estronsulfamat (2,0 g), gelöst in Dichlormethan (70 ml), wurde analog Beispiel 3 mit Boc-Anhydrid (2,5 g) in Gegenwart von Triethylamin (0,8 ml) und p-Dimethylaminopyridin (0,7 g) verestert. Die Reaktion war nach einer Stunde bei +23 °C beendet. Das nach Aufarbeitung erhaltene Produkt wurde an Kieselgel chromatographiert (Eluent: Dichlormethan/Ethylacetat; 7/3; v/v) und dann aus Aceton/n-Hexan umkristallisiert.
Fp.: 166-169 °C (Aceton/n-Hexan)

### Beispiel 5

### 17-Hydroxy-19-nor-17α-pregna-1,3,5(10)-trien-20-in-3-yl-(N-acetyl)sulfamat

17-Hydroxy-19-nor-17α-pregna-1,3,5(10)-trien-20-in-3-yl-sulfamat (2,0 g) wurde in Pyridin gelöst (50 ml). Man gab zu der Lösung Acetanhydrid (50 ml) und rührte das Gemisch 2 Stunden bei +23 °C. Aufarbeitung entsprechend Beispiel 1 ergab die Titelverbindung, die aus Aceton umkristallisiert wurde.
Fp.: 218-221 °C (Aceton)

### Beispiel 6

### 16α,17β-Dihydroxy-estra-1,3,5(10)-trien-3-yl-(N,N-dimethylcarbamoyl)sulfamat

16α,17β-Bis-(t-butyl-dimethyl)silyloxy-estra-1,3,5(10)-trien-3-ol (1,98 g) löste man in Dichlormethan (50 ml) und Triethylamin (4,9 ml). Nach Zugabe von p-Dimethylaminopyridin (0,22 g) und Dimethylcarbamoylchlorid (3,3 ml) wurde das Reaktionsgemisch 20 Stunden bei +23 °C gerührt. Danach wurde die Lösung nacheinander mit verdünnter Salzsäure, mit Wasser, gesättigter wäßriger Natriumhydrogencarbonatlösung und Wasser gewaschen, über wasserfreiem Natriumsulfat getrocknet und im Vakuumrotationsverdampfer eingeengt. Den Rückstand nahm man in einem Gemisch (75 ml) aus Essigsäure, Wasser und Tetrahydrofuran (3/1/1; v/v/v) auf. Nach 60stündigem Stehen bei +23 °C wurde das Gemisch mit gesättigter wäßriger Natriumhydrogencarbonatlösung neutralisiert und mit Dichlormethan extrahiert. Die vereinigten Extrakte wurden mit Wasser gewaschen und über wasserfreiem Natriumsulfat getrocknet und im Vakuumrotationsverdampfer eingeengt. Man erhielt die Titelverbindung als eine amorphe weiße Masse.

### Beispiel 7

### 17-Oxo-estra-1,3,5(10)-trien-3-yl-[N-methyl-N-(N'-methyl)sulfamoyl]sulfamat

Zu einer Lösung von Estron (3,0 g) in Dichlormethan (1,2 1) und Triethylamin (28,5 ml) ließ man N-Methylsulfamoylchlorid (3 ml) zutropfen. Man rührte das Gemisch 1,5 Stunden bei +23 °C, zersetzte dann mit Wasser (200 ml), wusch die organische Phase nacheinander mit verdünnter Salzsäure (1/1 v/v), Wasser, gesättigter wäßriger Natriumhydrogencarbonatlösung und Wasser, trocknete die organische Phase mit wasserfreiem Natriumsulfat und engte diese im Vakuumrotationsverdampfer ein. Das erhaltene Rohprodukt reinigte man durch Chromatographie an Kieselgel (Eluent: Toluol/Chloroform/Methanol; 80/15/5; v/v/v). Nach Umkristallisation aus Aceton/n-Hexan wurde die Titelverbindung erhalten.
Fp.: 179-185 °C (Aceton/n-Hexan)

### Beispiel 8

### 17-Oxo-estra-1,3,5(10)-trien-3-yl-(N-t-butyl-N-t-butoxycarbonyl)sulfamat

Estronsulfamat (2,0 g), gelöst in Dichlormethan (70 ml), wurde analog Beispiel 3 mit Boc-Anhydrid (2,5 g) in Gegenwart von Triethylamin (0,8 ml) und p-Dimethylaminopyridin (0,7 g) verestert, wobei man das Reaktionsgemisch über Nacht bei +23 °C stehen ließ. Das nach Aufarbeitung erhaltene Produkt wurde an Kieselgel chromatographiert (Eluent: Dichlormethan/Ethylacetat; 7/3; v/v) und dann aus Aceton/n-Hexan umkristallisiert.
Fp.: 162-167 °C (Aceton/n-Hexan)

### Beispiel 9

### 17β-Hydroxy-estra-1,3,5(10)-trien-3-yl-(N-acetyl)sulfamat (J 1045)

17-Oxo-estra-1,3,5(10)-trien-3-yl-(N-acetyl)sulfamat (1,0 g), hergestellt nach Beispiel 1, wurde bei 0 °C in einem Gemisch aus Tetrahydrofuran (100 ml) und Methanol (100 ml) mit Natriumborhydrid (0,68 g) reduziert. Nach Neutralisation der Reaktionslösung mit Essigsäure (2 ml) wurde diese im Vakuumrotationsverdampfer zur Trockene eingeengt. Den Rückstand nahm man in einem Gemisch aus Wasser (150 ml) und Ethylacetat (150 ml) auf. Die organische Phase wurde abgetrennt, mit Wasser gewaschen, mit wasserfreiem Natriumsulfat getrocknet und im Vakuumrotationsverdampfer eingeengt. Den Rückstand kristallisierte man aus Aceton/n-Hexan um, wobei die Titelverbindung erhalten wurde.
Fp.: 198-200 °C (Aceton/n-Hexan)

## Patentansprüche

1. Sulfamat-Derivate der allgemeinen Formel I 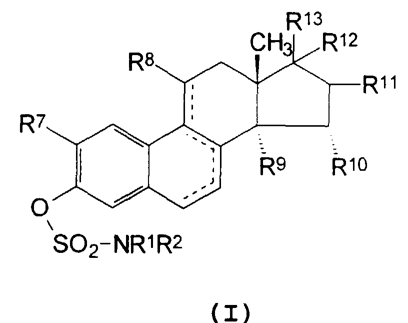 worin
R¹ eine -CO-R³-, -CO-OR⁴-, -CO-NR⁵R⁶- -SO₂-R⁴ oder -SO₂-NR⁵R⁶ -Gruppe ist,
worin
R³ ein Wasserstoffatom ist oder die Bedeutung von R⁴ hat,
R⁴ einen C₁-C₅-Alkyl-, einen C₂-C₅-Alkenylrest, einen C₃-C₆-Cycloalkylrest oder einen Arylrest mit bis zu 9 Kohlenstoffatomen darstellt,
R⁵ und R⁶ unabhängig voneinander ein Wasserstoffatom, einen C₁-C₅-Alkylrest, einen Arylrest mit bis zu 9 Kohlenstoffatomen oder zusammen mit dem N-Atom einen Polymethyleniminorest mit 3 bis 6 Kohlenstoffatomen oder einen Morpholinorest bedeuten,
R² ein Wasserstoffatom oder ein physiologisch verträgliches Metall ist oder die Bedeutung von R⁴ hat,
R⁷ und R⁸ unabhängig voneinander ein Wasserstoffatom, eine Hydroxygruppe oder einen C₁-C₅-Alkoxyrest darstellen,
R⁹ und R¹⁰ jeweils ein Wasserstoffatom oder zusammen eine Methylengruppe bedeuten,
R¹¹, R¹² und R¹³ unabhängig voneinander ein Wasserstoffatom oder eine Hydroxygruppe, welche gegebenenfalls mit physiologisch verträglichen anorganischen oder organischen Säuren verestert ist, darstellen,
oder R¹² oder R¹³ ein Alkinylrest mit bis zu 5 Kohlenstoffatomen ist,
die Ringe B und C gegebenenfalls eine oder zwei Doppelbindungen enthalten
und R⁸, R¹¹ und R¹² unabhängig voneinander α- oder β-ständig angeordnet sind.

2. Verfahren zur Herstellung der Sulfamat-Derivate nach Anspruch 1, dadurch gekennzeichnet, daß man in an sich bekannter Weise
a) geeignete Estra-1,3,5(10)-trien-3-yl-sulfamat-Derivate, welche am Stickstoffatom des Sulfamatrestes mindestens ein Wasserstoffatom tragen, mit einer geeigneten aktivierten Carbonsäure, Carbamidsäure, Sulfonsäure oder Amidosulfonsäure
oder
b) geeignete 3-Hydroxy-estra-1,3,5(10)-trien-Derivate mit einer geeigneten aktivierten N-Acyl-amidosulfonsäure, N-Sulfonylamidosulfonsäure oder N-Amidosulfonyl-amidosulfonsäure
jeweils gegebenenfalls in Gegenwart einer Base umsetzt, die so erhaltenen Produkte gegebenenfalls in geeigneter Weise weiter umsetzt und gegebenenfalls die so erhaltenen Produkte in physiologisch verträgliche Metallsalze überführt.

3. Pharmazeutische Zusammensetzungen, enthaltend mindestens ein Sulfamat-Derivat nach Anspruch 1, gegebenenfalls zusammen mit pharmazeutisch verträglichen Hilfs- und Trägerstoffen.

## Claims

1. Sulfamate derivatives of the general formula I 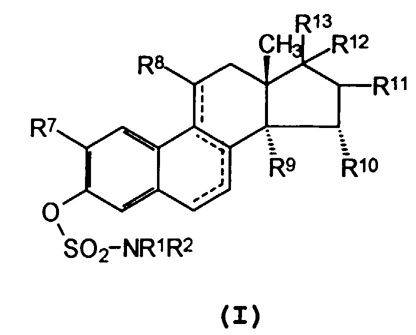 wherein
R¹ is a -CO-R³, -CO-OR⁴, -CO-NR⁵R⁶, -SO₂-R⁴ or -SO₂-NR⁵R⁶ group,
in which
R³ is a hydrogen atom or as defined in R⁴,
R⁴ represents a C₁-C₅ alkyl residue, a C₂-C₅ alkenyl residue, a C₃-C₆ cycloalkyl residue or an aryl residue containing up to 9 carbon atoms,
R⁵ and R⁶ are independent of each other and represent a hydrogen atom, a C₁-C₅ alkyl residue, an aryl residue containing up to 9 carbon atoms, or, together with the N atom, represent a polymethylene imino residue containing 3 to 6 carbon atoms, or a morpholino residue,
R² is a hydrogen atom or a physiologically tolerable metal, or is as defined in R⁴,
R⁷ and R⁸ are independent of each other and represent a hydrogen atom, a hydroxy group or a C₁-C₅ alkoxy residue,
R⁹ and R¹⁰ each represent a hydrogen atom, or together represent a methylene group,
R¹¹, R¹² and R¹³ are independent of each other and represent a hydrogen atom or a hydroxy group that may optionally be esterified with physiologically tolerable inorganic or organic acids, or R¹² or R¹³ is an alkinyl residue containing up to 5 carbon atoms,
rings B and C may optionally contain one or two double bonds,
and R⁸, R¹¹ and R¹² are independently placed either at the α- or β- position.

2. A method for the production of the sulfamate derivatives according to Claim 1, characterized in that
a) suitable estra-1,3,5(10)-triene-3-yl sulfamate derivatives that carry at least one hydrogen atom at the nitrogen atom of their sulfamate residue, are reacted with a suitable activated carboxylic acid, carbamic acid, sulfonic acid or amidosulfonic acid or
b) suitable 3-hydroxy-estra-1,3,5(10)-triene derivatives are reacted with a suitable activated N-acyl amidosulfonic acid, N-sulfonyl amidosulfonic acid or N-amidosulfonyl amidosulfonic acid,
in a generally known way, optionally in the presence of a base, that the products obtained are further reacted in a suitable way, and that the products thus obtained are optionally converted into physiologically tolerable metal salts.

3. Pharmaceutical preparations that contain at least one sulfamate derivative according to Claim 1, optionally combined with pharmaceutically acceptable adjuvants and substrates.

## Revendications

1. Dérivé sulfamate de formule générale I 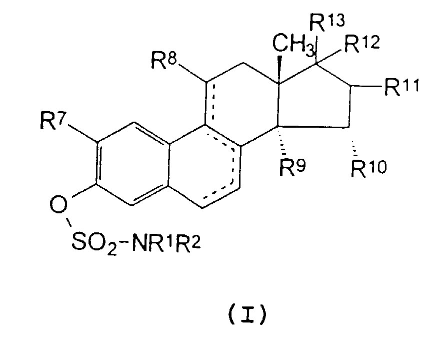 dans laquelle
R¹ représente un groupe -CO-R³, -CO-OR⁴, -CO-NR⁵R⁶, -SO₂-R⁴ ou -SO₂-NR⁵R⁶,
où
R³ représente un atome d'hydrogène ou a la même signification que R⁴,
R⁴ représente un reste C₁-C₅-alkyle, un reste C₂-C₅-alcényle, un reste C₃-C₆-cycloalkyle ou un reste aryle présentant jusqu'à 9 atomes de carbone,
R⁵ et R⁶ indépendamment les uns des autres, représentent un atome d'hydrogène, un reste C₁-C₅-alkyle, un reste aryle présentant jusqu'à 9 atomes de carbone ou bien ensemble avec l'atome d'azote représentent un reste polyméthylèneimino ayant de 3 à 6 atomes de carbone ou un reste morpholino,
R² représente un atome d'hydrogène ou bien un métal physiologiquement acceptable ou a la même signification que R⁴,
R⁷ et R⁸ indépendamment les uns des autres représentent un atome d'hydrogène, un groupe hydroxyle ou un reste C₁-C₅-alcoxy,
R⁹ et R¹⁰ représentent respectivement un atome d'hydrogène ou forment ensemble un groupe méthylène,
R¹¹, R¹² et R¹³ représentent indépendamment les uns des autres un atome d'hydrogène ou un groupe hydroxyle, lequel est éventuellement estérifié par un acide organique ou minéral physiologiquement acceptable,
ou bien R¹² ou R¹³ représente un reste un reste alcynyle ayant jusqu'à 5 atomes de carbone,
les cycles B et C contenant éventuellement une ou deux doubles liaisons, et les radicaux R⁸, R¹¹ et R¹² étant indépendamment les uns des autres en position α- ou β-.

2. Procédé pour la préparation de dérivés sulfamate selon la revendication 1, caractérisé en ce que, de façon conventionnelle,
(a) l'on fait réagir un dérivé approprié estra-1,3,5(10)-trièn-3-yl-sulfamate, lequel porte sur l'atome d'azote du reste sulfamate au moins un atome d'hydrogène, avec un acide carboxylique, un acide carbamique, un acide sulfonique ou un acide amidosulfonique activé approprié, ou bien
(b) l'on fait réagir un dérivé 3-hydroxy-estra-1,3,5(10)-triène avec un acide N-acyl-amidosulfonique, un acide N-sulfonylamidosulfonique ou un acide N-amidosulfonylamidosulfonique activé approprié,
dans chaque cas éventuellement en présence d'une base, les produits résultants étant ensuite éventuellement mis à réagir de façon appropriée et les produits ainsi obtenus étant éventuellement convertis en sels métalliques physiologiquement acceptables.

3. Composition pharmaceutique contenant au moins un dérivé sulfamate selon la revendication 1, éventuellement en association avec un additif ou un véhicule pharmaceutiquement acceptable.
